# FASCICULE DE BREVET EUROPEEN

(11) **EP 1 451 189 B1**
(45) Date de publication et mention de la délivrance du brevet: **25.05.2005**
(21) Numéro de dépôt: 02803453.6
(22) Date de dépôt: 21.11.2002
(51) Int. Cl.: C07D 471/08, A61K 31/55, A61P 25/00

(54) **DERIVES DE 4-(1,3,4-THIADIAZOL-2-YL)-1,4-DIAZABICYCLO- 3.2.2]NONANE, LEUR PREPARATION ET LEUR APPLICATION EN THERAPEUTIQUE**
4-(1,3,4-THIADIAZOLYL-2-YL)-1,4-DIAZABIZYKLO- 3.2.2 NONANDERIVATE, IHRE HERSTELLUNG UND IHRE THERAPEUTISCHE VERWENDUNG
4-(1,3,4-THIADIAZOL-2-YL)-1,4-DIAZABICYCLO- 3.2.2]NONANE DERIVATIVES, PREPARATION AND THERAPEUTIC USE THEREOF

(30) Priorité: 23.11.2001 FR 0115154
(43) Date de publication de la demande: 01.09.2004
(73) Titulaire: Sanofi-Aventis, 75013 Paris (FR)
(72) Inventeur: GALLI, Frédéric, F-92420 Vaucresson (FR); LECLERC, Odile, F-91300 Massy (FR); LOCHEAD, Alistair, F-94220 Charenton le Pont (FR)
(74) Mandataire: Ludwig, Jacques
(86) Numéro de dépôt international: PCT/FR2002/003986
(87) Numéro de publication internationale: WO 2003/044020

(56) Documents cités:
- EP-A- 0 307 140
- WO-A-00/34279
- WO-A-01/92260
- US-A- 5 478 939

## Description

Dérivés de 4-(1,3,4-thiadiazol-2-yl)-1,4-diazabicyclo-[3.2.2]nonane, leur préparation et leur application en thérapeutique.

La présente invention a pour objet des composés, ligands des récepteurs nicotiniques, utiles dans le traitement ou la prévention des désordres liés à un dysfonctionnement des récepteurs nicotiniques, notamment au niveau du système nerveux central.

Les composés de la présente invention répondent à la formule générale (I) 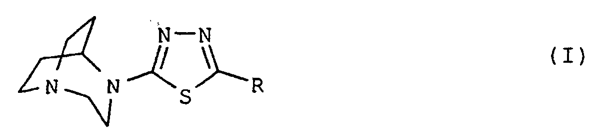 dans laquelle R représente un groupe (C₃-C₆)cycloalkyle ou un groupe phényle éventuellement substitué par un ou plusieurs groupes choisis parmi un atome d'halogène, un groupe (C₁-C₆)alkyle, (C₁-C₆)alcoxy, nitro, amino, dialkyl(C₁-C₃)amino, trifluorométhoxy, trifluorométhyle, cyano, hydroxy, méthylènedioxy, ou un groupe pipéridin-1-yle, ou morpholin-4-yle, ou pyrrolidin-1-yle, ou azétidin-1-yle, ou azépin-1-yle, ou pyridinyle, ou thiényle, ou pyrazinyle, ou furyle, ou benzofuryle, ou benzothiényle, ou indolyle, ou pyrimidinyle, ou isoxazolyle, ou phénoxazinyle, ou phénoxathiinyle, ou dibenzofuryle ou dibenzothiényle ou pyrrolyle, ou naphtyle, chacun de ces groupes pouvant éventuellement être substitué par un ou plusieurs groupes choisis parmi un atome d'halogène, un groupe (C₁-C₆)alkyle, (C₁-C₆)alcoxy, trifluorométhoxy, trifluorométhyle, nitro, cyano, hydroxy, amino, (C₁-C₃)dialkylamino, ou (C₃-C₈)cycloalkylamino.

Un sous ensemble de composés préférés est celui des composés de formule générale (I) dans laquelle R représente un groupe phényle substitué par un ou plusieurs atomes d'halogène, par un ou plusieurs groupes (C₁-C₆)alkyle, (C₁-C₆)alcoxy, nitro, amino, trifluorométhoxy, trifluorométhyle, cyano, hydroxy ou méthylènedioxy, ou un groupe pyridinyle, ou un groupe thiényle éventuellement substitué par un atome d'halogène, ou un groupe pyrazinyle.

Les composés de l'invention peuvent exister à l'état de bases ou de sels d'addition à des acides.

Conformément à l'invention on peut préparer les composés de formule générale (I) selon le schéma suivant, en faisant réagir le 1,4-diazabicyclo[3.2.2]nonane de formule (II) avec un composé de formule générale (III) dans laquelle R est tel que défini ci-dessus.

On peut ainsi effectuer une réaction de substitution nucléophile en présence d'une base forte telle que le carbonate de césium ou la triéthylamine. On peut aussi effectuer une réaction de couplage de type Buchwald (*J*. *Org. Chem.* 1997, **62,** 6066-6068) en présence d'un catalyseur au palladium tel que l'acétate de palladium, le tris(dibenzylidèneacétone)dipalladium (0), etc, d'un ligand de complexation tel que la triphénylphosphine, la tributylphosphine ou le 2,2'-bis(diphénylphosphino)-1,1'-binaphtyle, et d'une base, par exemple organique telle que le t-butoxyde de sodium, ou minérale telle que le carbonate de césium, ou toute autre réaction de couplage similaire.

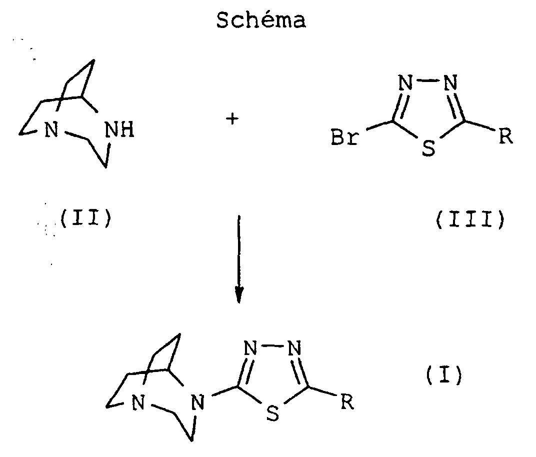

La préparation du 1,4-diazabicyclo[3.2.2]nonane est décrite dans *J. Med. Chem.* 1993, **36,** 2311-2320.

Les composés de formule générale (III) sont disponibles dans le commerce ou sont accessibles par des méthodes décrites dans la littérature, par exemple dans *J. Het. Chem.* 1983, 73.

L'exemple qui va suivre illustre la préparation des composés de l'invention. Les microanalyses élémentaires, et les spectres I.R. et R.M.N., ainsi que, dans certains cas, les spectres de diffraction aux rayons X, confirment les structures des composés obtenus.
Le numéro indiqué entre parenthèses dans le titre de l'exemple correspond à ceux de la 1ère colonne du tableau donné plus loin.
Dans les noms des composés, le tiret "-" fait partie du mot, et le tiret "_" ne sert que pour la coupure en fin de ligne ; il est à supprimer en l'absence de coupure, et ne doit être remplacé ni par un tiret normal ni par un espace.

### Exemple (Composé N°1).

### Bromhydrate de 4-[(5-phényl)-1,3,4-thiadiazol-2-yl)-1,4-diazabicyclo[3.2.2]nonane 2:1.

Dans un ballon de 25 ml on introduit successivement 0,5 g (4 mmoles) de 1,4-diazabicyclo[3.2.2]nonane, 1 g (4 mmoles) de 2-bromo-5-phényl-1,3,4-thiadiazole et 0,6 ml (4,4 mmoles) de triéthylamine en solution dans 15 ml de tétrahydrofurane sec, et on chauffe le mélange au reflux pendant 20 h.
On évapore le solvant sous pression réduite et on purifie le résidu par chromatographie sur colonne de gel de silice en éluant avec un mélange 93/7/0,7 de chloroforme, méthanol et ammoniaque.
On obtient 0,25 g de produit dont on fait le dibromhydrate par addition d'une solution d'acide bromhydrique à 33% dans l'acide acétique. On collecte les cristaux obtenus par filtration.

On obtient 0,21 g de dibromhydrate.
Point de fusion : 297-300°C.

Le tableau qui suit illustre les structures chimiques et les propriétés physiques de quelques composés de l'invention.
Dans la colonne "Sel", "-" désigne un composé à l'état de base et "HBr" désigne un bromhydrate. Les rapports molaires acide:base sont indiqués en regard.
Dans la colonne "F (°C)", "(d)" désigne un point de fusion avec décomposition.

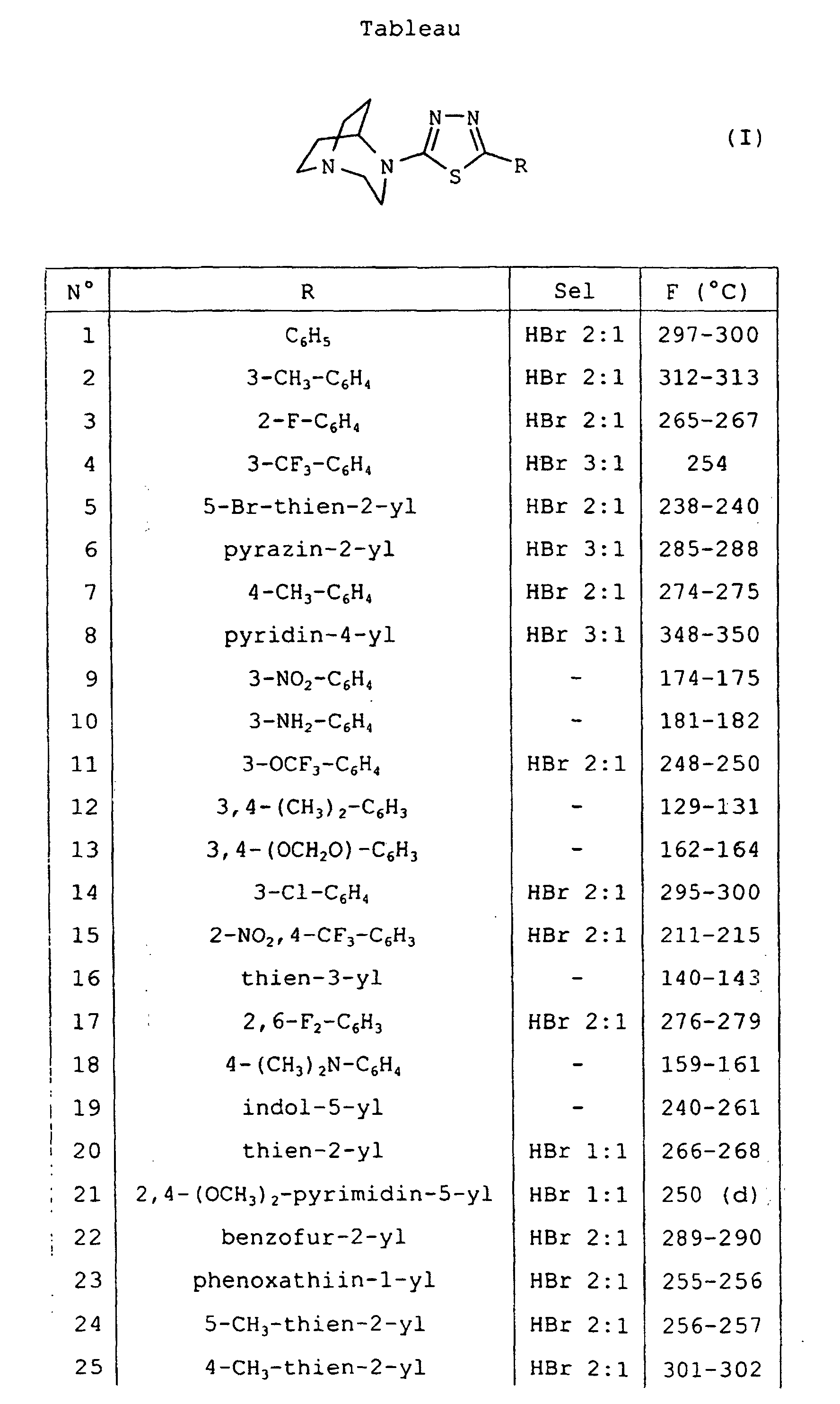

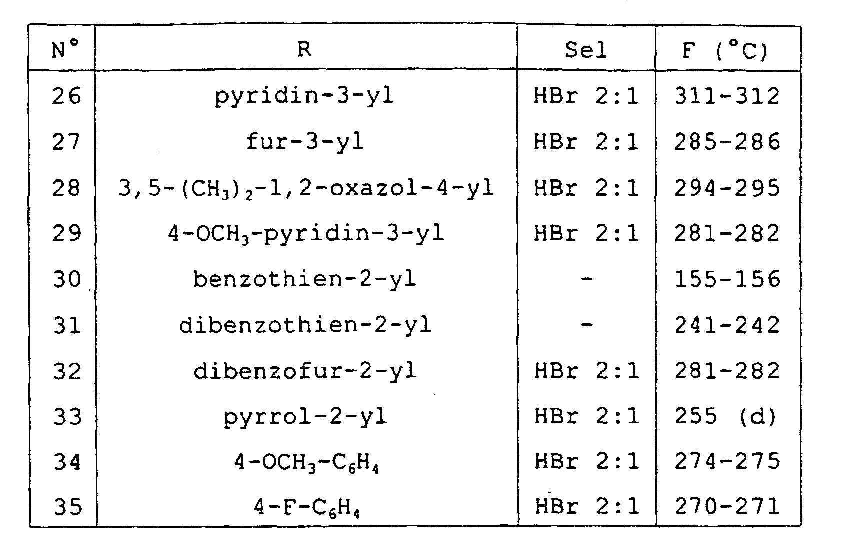

Les composés de l'invention ont été étudiés quant à leur affinité vis-à-vis des récepteurs nicotiniques contenant la sous unité α₇, selon les méthodes décrites par Mark et Collins dans *J. Pharmacol. Exp. Ther*. 1982, **22,** 564 et par Marks et coll. dans *Mol. Pharmacol*. 1986, **30,** 427.
On décapite des rats mâles OFA de 150 à 200 g, on prélève rapidement la totalité du cerveau, on l'homogénéise à l'aide d'un broyeur Polytron™ dans 15 volumes d'une solution de sucrose à 0,32 M à 4°C, puis on le centrifuge à 1000 G pendant 10 min. On élimine le culot et on centrifuge le surnageant à 8000 G pendant 20 min à 4°C. On récupère le culot et on l'homogénéise à l'aide d'un broyeur Polytron ™ dans 15 volumes d'eau bidistillée à 4°C, puis on le centrifuge à 8000 G pendant 20 min. On élimine le culot et on centrifuge le surnageant et la couche de peau ("buffy coat") à 40000 G pendant 20 min. On récupère le culot, on le remet en suspension avec 15 volumes d'eau bidistillée à 4°C et on le centrifuge encore une fois à 40000 G pendant 20 min avant de le conserver à -80°C.

Le jour de l'expérience on décongèle lentement le tissu et on le met en suspension dans 5 volumes de tampon. On préincube 150 µl de cette suspension membranaire à 37°C pendant 30 min, à l'obscurité, en présence ou en absence du composé à tester. Puis les membranes sont incubées pendant 60 min à 37°C, à l'obscurité, en présence de 50 µl de [³H]α-bungarotoxine à 1 nM dans un volume final de 250 µl de tampon HEPES 20 mM. On arrête la réaction par filtration sur des filtres Whatman GF/C™ préalablement traités pendant 3 heures avec de la polyéthylènimine à 0,05%. On rince les filtres avec deux fois 5 ml de tampon à 4°C et on mesure la radioactivité retenue sur chaque filtre par scintigraphie liquide. On détermine la liaison non spécifique en présence de α-bungarotoxine à 1 µM finale ; la liaison non spécifique représente environ 60% de la liaison totale récupérée sur le filtre. Pour chaque concentration de composé étudié on détermine le pourcentage d'inhibition de la liaison spécifique de [³H]α-bungarotoxine, puis on calcule la CI₅₀, concentration de composé qui inhibe 50% de la liaison spécifique.
Les CI₅₀ des composés de l'invention les plus affins se situent entre 0,001 et 0,5 µM.

Les résultats qui précèdent montrent que les composés de l'invention sont des ligands pour les sous unités α₇ du récepteur nicotinique.

Les résultats des essais suggèrent l'utilisation des composés dans le traitement ou la prévention des désordres liés à un dysfonctionnement des récepteurs nicotiniques, notamment au niveau du système nerveux central.

Ces désordres comprennent les altérations cognitives, plus spécifiquement mnésiques, mais également attentionnelles, liées à la maladie d'Alzheimer, au vieillissement pathologique (Age Associated Memory Impairment,AAMI), au syndrome Parkinsonien, à la trisomie 21 (Down's syndrome), au syndrome alcoolique de Korsakoff, aux démences vasculaires (multi-infarct dementia, MDI).
Les composés de l'invention pourraient également être utiles dans le traitement des troubles moteurs observés dans la maladie de Parkinson ou d'autres maladies neurologiques telles que la chorée de Huntington, le syndrome de Tourette, la dyskinésie tardive et l'hyperkinésie.
Les composés de l'invention peuvent également constituer un traitement curatif ou symptomatique des pathologies neurodégénératives aiguës telles que les accidents vasculaires cérébraux et les épisodes hypoxiques cérébraux, ainsi que des pathologies neurodégénératives chroniques telles que la maladie d'Alzheimer. Ils peuvent encore être utilisés dans les cas de pathologies psychiatriques : schizophrénie, dépression, anxiété, attaques de panique, comportements compulsifs et obsessionnels.
Ils peuvent prévenir les symptômes dus au sevrage au tabac, à l'alcool, aux différentes substances induisant une dépendance, telles que cocaïne, LSD, cannabis, benzodiazépines.

C'est pourquoi la présente invention a également pour objet des compositions pharmaceutiques contenant une dose efficace d'au moins un composé selon l'invention, à l'état de base ou de sel ou de solvat pharmaceutiquement acceptable, et en mélange, le cas échéant, avec des excipients convenables.

Lesdits excipients sont choisis selon la forme pharmaceutique et le mode d'administration souhaité.

Les compositions pharmaceutiques selon l'invention peuvent ainsi être destinées à l'administration orale, sublinguale, sous-cutanée, intramusculaire, intraveineuse, topique, intratrachéale, intranasale, transdermique, rectale, intraocculaire.

Les formes unitaires d'administration peuvent être, par exemple, des comprimés, des gélules, des granules, des poudres, des solutions ou suspensions orales ou injectables, des timbres transdermiques ("patch"), des suppositoires. Pour l'administration topique on peut envisager des pommades, lotions et collyres.
Lesdites formes unitaires sont dosées pour permettre une administration journalière de 0,01 à 20 mg de principe actif par kg de poids corporel, selon la forme galénique.

Pour préparer des comprimés on ajoute au principe actif, micronisé ou non, un véhicule pharmaceutique qui peut être composé de diluants, comme par exemple le lactose, la cellulose microcristalline, l'amidon, et des adjuvants de formulation comme des liants, (polyvinylpyrrolidone, hydroxypropylméthylcellulose, etc), des agents d'écoulement comme la silice, des lubrifiants comme le stéarate de magnésium, l'acide stéarique, le tribehenate de glycerol, le stéarylfumarate de sodium. Des agents mouillants ou tensioactifs tels que le laurylsulfate de sodium peuvent aussi être ajoutés.
Les techniques de réalisation peuvent être la compression directe, la granulation sèche, la granulation humide ou la fusion à chaud.
Les comprimés peuvent être nus, dragéifiés, par exemple par du saccharose, ou enrobés avec divers polymères ou autres matières appropriées. Il peuvent être conçus pour permettre une libération rapide, retardée ou prolongée du principe actif grâce à des matrices polymères ou à des polymères spécifiques utilisés dans l'enrobage.

Pour préparer des gélules on mélange le principe actif avec des véhicules pharmaceutiques secs (simple mélange, granulation sèche ou humide, ou fusion à chaud), liquides ou semi-solides.
Les gélules peuvent être dures ou molles, pelliculées ou non, de manière à avoir une activité rapide, prolongée ou retardée (par exemple pour une forme entérique).

Une composition sous forme de sirop ou d'élixir ou pour l'administration sous forme de gouttes peut contenir le principe actif conjointement à un édulcorant, de préférence acalorique, du méthylparaben ou du propylparaben comme antiseptique, un agent de sapidité et un colorant.

Les poudres et granules dispersibles dans de l'eau peuvent contenir le principe actif en mélange avec des agents de dispersion ou des agents mouillants, ou des agents dispersants comme la polyvinylpyrrolidone, de même qu'avec des édulcorants et des agents correcteurs de goût.

Pour l'administration rectale, on recourt à des suppositoires préparés avec des liants fondant à la température rectale, par exemple du beurre de cacao ou des polyéthylèneglycols.

Pour une administration parentérale, on utilise des suspensions aqueuses, des solutions salines isotoniques ou des solutions stériles injectables contenant des agents de dispersion et/ou des mouillants pharmacologiquement compatibles, par exemple le propylèneglycol ou le butylèneglycol.

Le principe actif peut être formulé également sous forme de microcapsules, éventuellement avec un ou plusieurs supports ou additifs, ou bien avec une matrice polymère ou avec une cyclodextrine (timbres transdermiques, formes à libération prolongée).

Les compositions topiques selon l'invention comprennent un milieu compatible avec la peau. Elles peuvent se présenter notamment sous forme de solutions aqueuses, alcooliques ou hydroalcooliques, de gels, d'émulsions eau-dans-huile ou huile-dans-eau ayant l'aspect d'une crème ou d'un gel, de microémulsions, d'aérosols, ou encore sous forme de dispersions vésiculaires contenant des lipides ioniques et/ou non ioniques. Ces formes galéniques sont préparées selon les méthodes usuelles des domaines considérés.

Enfin, les compositions pharmaceutiques selon l'invention peuvent contenir, à côté d'un composé de formule générale (I), d'autres principes actifs qui peuvent être utiles dans le traitement des troubles et maladies indiqués ci-dessus.

## Revendications

1. Composé répondant à formule générale (I) 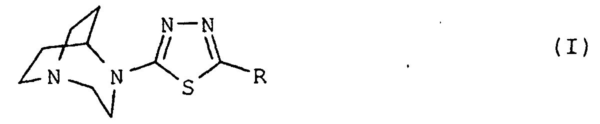 dans laquelle R représente un groupe (C₃-C₆)cycloalkyle ou un groupe phényle éventuellement substitué par un ou plusieurs groupes choisis parmi un atome d'halogène, un groupe (C₁-C₆)alkyle, (C₁-C₆)alcoxy, nitro, amino, dialkyl(C₁-C₃)amino, trifluorométhoxy, trifluorométhyle, cyano, hydroxy, méthylènedioxy, ou un groupe pipéridin-1-yle, ou morpholin-4-yle, ou pyrrolidin-1-yle, ou azétidin-1-yle, ou azépin-1-yle, ou pyridinyle, ou thiényle, ou pyrazinyle, ou furyle, ou benzofuryle, ou benzothiényle, ou indolyle, ou pyrimidinyle, ou isoxazolyle, ou phénoxazinyle, ou phénoxathiinyle, ou dibenzofuryle ou dibenzothiényle ou pyrrolyle, ou naphtyle, chacun de ces groupes pouvant éventuellement être substitué par un ou plusieurs groupes choisis parmi un atome d'halogène, un groupe (C₁-C₆)alkyle, (C₁-C₆)alcoxy, trifluorométhoxy, trifluorométhyle, nitro, cyano, hydroxy, amino, (C₁-C₃)dialkylamino, ou (C₃-C₈)cycloalkylamino, à l'état de base ou de sel d'addition à un acide.

2. Composé selon la revendication 1, **caractérisé en ce que** R représente un groupe phényle substitué par un ou plusieurs atomes d'halogène, par un ou plusieurs groupes (C₁-C₆)alkyle, (C₁-C₆)alcoxy, nitro, amino, trifluorométhoxy, trifluoro-méthyle, cyano, hydroxy ou méthylènedioxy, ou un groupe pyridinyle, ou un groupe thiényle éventuellement substitué par un atome d'halogène, ou un groupe pyrazinyle.

3. Médicament, **caractérisé en ce qu'**il consiste en un composé selon l'une des revendications 1 et 2.

4. Composition pharmaceutique, **caractérisée en ce qu'**elle contient un composé selon l'une des revendication 1 et 2, associé à un excipient.

## Patentansprüche

1. Verbindung der allgemeinen Formel: 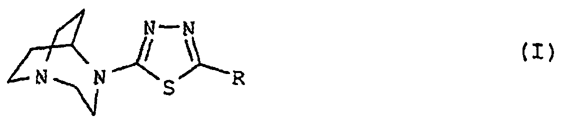 in der R eine (C₃-C₆)-Cycloalkylgruppe oder eine Phenylgruppe, die gegebenenfalls durch eine oder mehrere Gruppen ausgewählt aus einem Halogenatom, einer (C₁-C₆)-Alkyl-, (C₁-C₆-)-Alkoxy-, Nitro-, Amino-, Di-(C₁-C₃)-alkyl-amino-, Trifluormethoxy-, Trifluormethyl-, Cyano-, Hydroxy-, oder Methylendioxy-Gruppe substituiert ist, oder eine Piperidin-1-yl-, oder Morpholin-4-yl-, oder Pyrrolidin-1-yl-, oder Azetidin-1-yl-, oder Azepin-1-yl-, oder Pyridinyl-, oder Thienyl-, oder Pyrazinyl-, oder Furyl-, oder Benzofuryl-, oder Benzothienyl-, oder Indolyl-, oder Pyrimidinyl-, oder Isoxazolyl-, oder Phenoxazinyl-, oder Phenoxathiinyl-, oder Dibenzofuryl-, oder Dibenzothienyl-, oder Pyrrolyl-, oder Naphthyl-Gruppe, wobei jede dieser Gruppen gegebenenfalls durch eine oder mehrere Gruppen ausgewählt aus einem Halogenatom, einer (C₁-C₆)-Alkyl-, (C₁-C₆)-Alkoxy-, Trifluormethoxy-, Trifluormethyl-, Nitro-, Cyano-, Hydroxy-, Amino-, Di-(C₁-C₃)-alkylamino-, oder (C₃-C₈)-Cycloalkylamino-Gruppe substituiert sein kann, bedeutet, in Form der Base oder eines Säureadditionssalzes.

2. Verbindung nach Anspruch 1, **dadurch gekennzeichnet, daß** R eine durch ein oder mehrere Halogenatome, durch eine oder mehrere (C₁-C₆)-Alkyl-, (C₁-C₆)-Alkoxy-, Nitro-, Amino-, Trifluormethoxy-, Trifluormethyl-, Cyano-, Hydroxy- oder Methylendioxy-Gruppen substituierte Phenylgruppe oder eine Pyridinylgruppe, oder eine Thienylgruppe, die gegebenenfalls durch ein Halogenatom oder eine Pyrazinylgruppe substituiert sind, bedeutet.

3. Arzneimittel, **dadurch gekennzeichnet, daß** es aus einer Verbindung nach einem der Ansprüche 1 und 2 besteht.

4. Pharmazeutische Zubereitung, **dadurch gekennzeichnet, daß** sie eine Verbindung nach einem der Ansprüche 1 und 2 in Kombination mit einem Hilfsstoff enthält.

## Claims

1. Compound corresponding to the general formula (I) 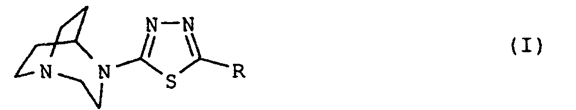 in which R represents a (C₃-C₆)cycloalkyl group or a phenyl group optionally substituted with one or more groups chosen from a halogen atom, or a (C₁-C₆)alkyl, (C₁-C₆)alkoxy, nitro, amino, di(C₁-C₃)alkylamino, trifluoromethoxy, trifluoromethyl, cyano, hydroxyl or methylenedioxy group or a 1-piperidyl, 4-morpholinyl, 1-pyrrolidinyl, 1-azetidinyl, 1-azepinyl, pyridyl, thienyl, pyrazinyl, furyl, benzofuryl, benzothienyl, indolyl, pyrimidinyl, isoxazolyl, phenoxazinyl, phenoxathiinyl, dibenzofuryl, dibenzothienyl, pyrrolyl or naphthyl group, each of these groups being optionally substituted with one or more groups chosen from a halogen atom and a (C₁-C₆)alkyl, (C₁-C₆)alkoxy, trifluoromethoxy, trifluoromethyl, nitro, cyano, hydroxyl, amino, di(C₁-C₃)alkylamino or (C₃-C₈)cycloalkylamino group, in base form or in the form of an acid-addition salt.

2. Compound according to Claim 1, **characterized in that** R represents a phenyl group substituted with one or more halogen atoms or with one or more (C₁-C₆)alkyl, (C₁-C₆)alkoxy, nitro, amino, trifluoromethoxy, trifluoromethyl, cyano, hydroxyl or methylenedioxy groups, or a pyridyl group, or a thienyl group optionally substituted with a halogen atom, or a pyrazinyl group.

3. Medicinal product, **characterized in that** it consists of a compound according to either of Claims 1 and 2.

4. Pharmaceutical composition, **characterized in that** it contains a compound according to either of Claims 1 and 2, combined with an excipient.
